# EUROPEAN PATENT SPECIFICATION

(11) **EP 0 357 092 B1**
(45) Date of publication and mention of the grant of the patent: **27.10.1993**
(21) Application number: 89118973.0
(22) Date of filing: 19.12.1984
(51) Int. Cl.: C07C 69/21, C07C 67/08

(54) **Preparation of diacetoxybenzylidene diacetates**
Herstellung von Diacetoxybenzylidendiacetaten
Préparation de diacétoxybenzylidène diacétates

(30) Priority: 26.12.1983 JP 251692/83; 22.06.1984 JP 128789/84
(43) Date of publication of application: 07.03.1990
(62) Divisional of application: 84308919.4
(73) Proprietor: KUREHA KAGAKU KOGYO KABUSHIKI KAISHA, Chuo-ku Tokyo 103 (JP)
(72) Inventor: Takita, Hitoshi, Nerima-ku Tokyo (JP); Kimura, Fumihiko, Shinjuku-ku Tokyo (JP); Noda, Sakuo, Nerima-ku Tokyo (JP); Mukaida, Yutaka, Iruma-gun Saitama-ken (JP); Nitta, Toyohiko, Shinjuku-ku Tokyo (JP); Kobayashi, Hidetoshi, Suginami-ku Tokyo (JP)
(74) Representative: Woods, Geoffrey Corlett

(56) References cited:
- US-A- 2 748 160
- DRUG METABOLISM AND DISPOSITION, vol. 7, no. 3, 1979, pages 155-161, The American Society for Pharmacology and Experimental Therapeutics, US; S. AGURELL et al.: "Chemical synthesis and biolocigal occurrence of carboxylic acid metabolites of delta-1(6)-tetrahydrocannabinol"

## Description

The present invention relates to a process to prepare 3,4- or 3,5-diacetoxybenzylidene diacetate represented by formula (I) or (I'): 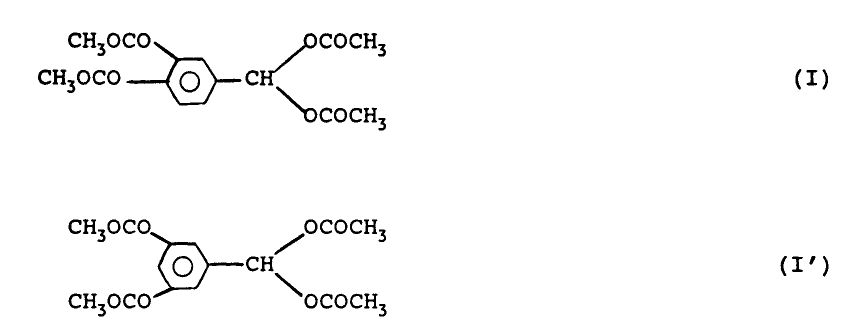
3,4-Dihydroxybenzaldehyde has recently attracted attention as an antitumour agent (JP-A-55-51018(1980)) and as an anti-inflammatory agent (JP-A-58-83619(1983)).

However, although dihydroxybenzaldehyde shows an excellent pharmacological activity in suppressing platelet aggregation and migration of leukocytes at a relatively low concentration in vitro, owing to the rapid metabolism thereof in the living body, it is necessary to administer a large amount thereof for a long time period for obtaining the effective effects of such pharmacological activity in vivo, and there are difficulties in administering thereof due to the stimulus action and the oxidizability of the aldehyde moiety thereof.

As a result of the present inventors' studies for developing a pharmaceutical agent which exhibits an effective pharmacological activity when administered to a living body even at a small dose rate while scarcely showing side effects, the present inventors have found the compounds represented by the formulae (I) and (I') can be synthesized by the reaction between a dihydroxybenzaldehyde and acetic acid anhydride.

Accordingly the present invention provides a process for the preparation of 3,4- or 3,5-diacetoxybenzylidene diacetate, which process comprises reacting 3,4- or 3,5-dihydroxybenzaldehyde with (CH₃CO)₂O in the presence of concentrated sulphuric acid while stirring or shaking for a time period of from one minute to five hours.

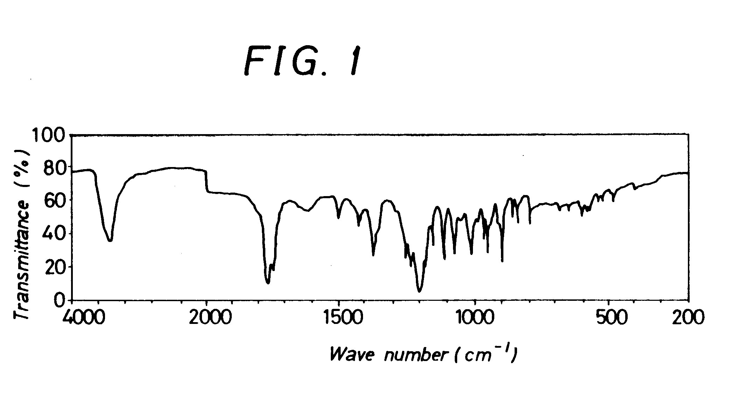

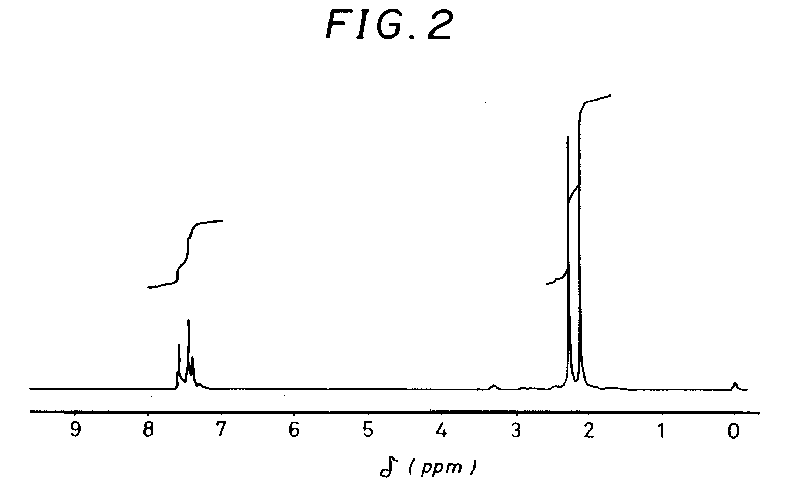

In the attached drawings, Figure 1 is the infrared absorption spectrum of the present substance No.1 according to the present invention, and Figure 2 is the nuclear magnetic resonance spectrum of the same substance (No.1).

The novel process for producing 3,4- or 3,5-diacetoxybenzylidene diacetate (hereinafter referred to as the present substance) is explained as follows. The present substance can be profitably synthesized in a high yield when acetic acid anhydride is reacted with 3,4- or 3,5-dihydroxybenzaldehyde in the presence of a strong acid, in particular concentrated sulfuric acid, as is shown by the following reaction formulae: 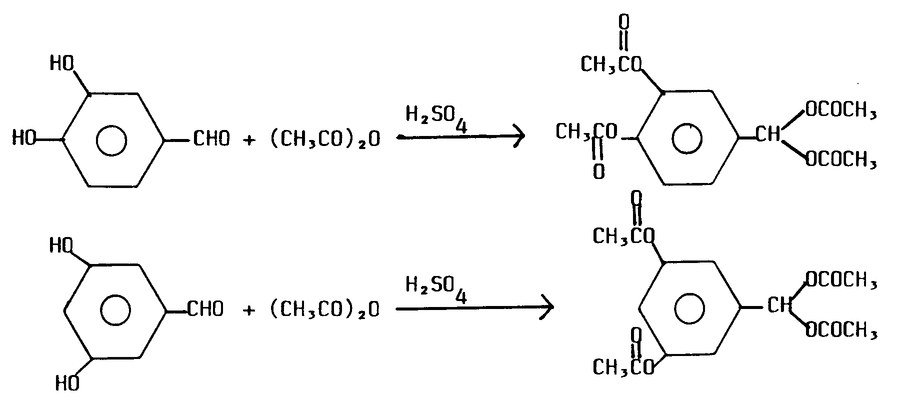
Typically, to one mole of 3,4- or 3,5-dihydroxybenzaldehyde, more than 3 moles of acetic acid anhydride are added and, after melting the anhydride at a temperature higher than the melting point of the anhydride and in the range of from room temperature to 100°C, a catalytic amount of concentrated sulfuric acid is rapidly added to the mixture while stirring or shaking the mixture. Then, the reaction exothermically proceeds to obtain a homogeneous reaction mixture as a solution. The reaction is completed within a time period of from one min to five hours, preferably from two min to one hour.

From the thus obtained reaction mixture, the present substance represented by the formula (I) or (I') can be isolated by one of known methods such as recrystallization, extraction and removal of the by-produced acetic acid by extraction, evaporation of the un-reacted anhydride of acetic acid or column-chromatography.

Each of the present substances showed an activity of suppressing the migration of leukocytes and an activity of inhibiting the proliferation of granuloma as the results of in vivo tests. In addition, each of the present substances is less toxic than the known substances such as 3,4-dihydroxybenzaldehyde and is effective at a smaller dose rate than that of the known substances such as dihydroxybenzaldehyde. Accordingly, each of the present substances has a pharmacological activity as an anti-inflammatory agent.

The mammalian toxicity and pharmacological properties of the present substances are explained as follows by the representative compound 3,4-diacetoxybenzylidene diacetate.

3,4-diacetoxybenzylidene diacetate is hereinafter referred to as the present substance No.1.

### (1) Acute mammalian toxicity

After dispersing present substance No.1 in an aqueous 0.2 % solution of carboxymethylcellulose, the aqueous dispersion was orally administered to each of male Jcl-ICR mice. As a result, LD₅₀ (acute, oral) of the tested substance was larger than 4,000 mg/kg.

On the other hand, LD₅₀ (acute, oral) of 3,4-dihydroxybenzaldehyde to male Jcl-ICR mice was 1503 mg/kg and accordingly, 3,4-diacetoxybenzylidene diacetate was found to be extremely low in acute mammalian toxicity.

### (2) Activity in suppressing the migration of leukocytes:

While using groups of male Donryu rats (six rats per group) and following the carboxymethylcellulose-pouch method (refer to Ishikawa et al. YAKUGAKU ZASSHI (Journal of the Pharmaceutical Society of Japan), 88, 1472, 1968), the extent of inhibition of migration of polymorphonuclear leukocytes to the site of inflammation by present substance No.1 was examined. The specimen (present substance No.1) was dispersed in an aqueous 0.2 % solution of carboxymethylcellulose and the dispersion was administered to each rat at a predetermined dose rate, and only the aqueous 0.2 % solution of carboxymethylcellulose was administered to each rat of control group. The test was carried out by injecting the aqueous 0.2 % solution of carboxymethyl-cellulose into the pouch formed in the body of the rat and after 6 hours of the injection, the number of polymorphonuclear leukocytes in the exudate into the pouch was counted. The results are shown in Table 1, and as are seen in Table 1, it was confirmed that present substance No.1 significantly suppressed the migration of polymorphonuclear leukocytes (PMN) to the site of inflammation.

**TABLE 1**

| Compound administered | Dose Rate (mg/kg) | Rate of suppression of migration of PMN (%) |
|---|---|---|
| Present Substance No.1 | 5 | 41.2 |
| | 10 | 48.0 |
| | 50 | 51.9 |
| Prednisolone | 5 | 37.5 |
| Indomethacin | 5 | 35.7 |
| 3,4-dihydroxy-benzaldehyde | 50 | 27.5 |

### (3) Activity in suppressing the proliferation of granuloma:

While using groups of male Donryu rats in the fifth week after birth (5 rats per group), the activity of present substance No.1 in suppressing the proliferation of granuloma was tested by the method of Fujimura (refer to OYOYAKURI (Pharmacometrics), 19 (3), 329, 1980).

After soaking sheets of filter paper of 13 mm in diameter and 28 mg in weight into an aqueous 2% solution of carboxymethylcellulose containing both dihydroxystreptomycin and penicillin, each of 10⁶ unit at the respective concentration of 0.1 mg/ml, each of the thus treated sheets was buried subcutaneously into the back of each rat under anaesthesia by ether. Present substance No.1 was dispersed in an aqueous 0.3 % solution of carboxymethylcellulose and the dispersion was administered orally to the thus treated rat after the rat had been awakened from the anaesthesia once a day for 10 days. After 11 days of the treatment, granuloma formed in the rat was removed, dried for 24 hours at 70°C and weighed. To the rats of control group, only the aqueous 0.3 % solution of carboxymethylcellulose was orally administered once a day for 10 days, both results being shown in Table 2.

As is seen in Table 2, 3,4-diacetoxybenzylidene diacetate significantly suppressed the proliferation of granulating tissue formed.

**TABLE 2**

| Compound Administered | Dose Rate (mg/kg/day) | Granuloma |
|---|---|---|
| | | Rate of Suppression (%) |
| Present Substance No. 1 | 5 | 42.8 |
| | 10 | 55.3 |
| | 50 | 61.4 |
| Indomethacin | 3 | 39.4 |
| Prednisolone | 3 | 42.2 |
| 3,4-dihydroxy-benzaldehyde | 50 | 35.6 |

As are seen in the above-mentioned test results, it can be recognised that the present substance has excellent activity in suppressing the proliferation of granuloma and in suppressing the migration of leukocytes and is extremely low in acute mammalian toxicity.

The present substance can be administered orally, intraintestinally or in the form of injection in one of the various forms of pharmaceutical formulation (so-called pharmaceutical composition) after being combined with pharmaceutically acceptable carrier(s) and/or adjuvant(s). The present substances may be used in combination or after being mixed together, and the present substance may be used after being combined with any other active ingredient for pharmaceutical use.

Since the present substance can be administered orally and parenterally, it can take any optional form of pharmaceutical compositions suitable for the route of administration. In addition, the present substance may be offered in the unit dose form, and as far as the pharmaceutical composition contains an effective amount of the present substance, the composition can take the various forms such as powder, granule, tablet, sugar-coated tablet, capsule, suppository, suspension, solution, emulsion, ampoule and injection.

Accordingly, it should be recognized that the pharmaceutical composition comprising the present substance can be formulated by application of any known means for formulation of a pharmacologically active agent.

In addition, the content of the present substance as an active ingredient in the above-mentioned pharmaceutical composition can be adjusted in a broad range of from 0.01 to 100% by weight, preferably in a range of from 0.1 to 70% by weight.

As has been stated, although the pharmaceutical composition comprising the present substance is orally or parenterally administered to human or mammals, the oral administration including sublingual administration is particularly preferable.

The parenteral administration includes subcutaneous-, intramuscular- and intravenous injection and instillation.

Since the dose rate of the present substance depends on the species, the sex, the age, the individual difference and the state of the disease of the patient to be administered therewith, there may be cases where an amount outside the following range is administered, however, in the cases where human being is the object of administration, the oral daily dose rate of one of the present substances is in a range of from 0.1 to 500 mg/kg body weight, preferably in a range of from 0.5 to 200 mg/kg body weight, more preferably in the range of from 3 to 100 mg/kg body weight, and the parenteral daily dose rate is in a range of from 0.01 to 200 mg/kg body weight, preferably in a range of from 0.1 to 100 mg/kg body weight, more preferably in the range of from 1.5 to 50 mg/kg body weight. The above-mentioned daily amount is divided equally into 1 to 4 portions, and the thus divided portion is administered at a time (one to four times per day).

The present invention will be explained more in detail while referring to the following non-limitative examples.

### EXAMPLE 1:

### Synthesis of 3,4-diacetoxybenzylidene diacetate (the present substance No. 1)

After introducing 30 g of protocatechualdehyde(3,4-dihydroxybenzaldehyde) and 92.4 g of acetic anhydride into a 200 ml-flask and adding one drop of concentrated sulfuric acid to the content of the flask, the flask was shaken to rapidly induce a beginning of an exothermic reaction, thereby obtaining a uniform liquid reaction mixture of red in colour. After shaking the flask for 3 min, the liquid reaction mixture was poured into 500 ml of water to form a colourless, powdery crude product as a precipitate. The amount of the crude product after collecting the precipitate by filtration and drying thereof was 68.0 g (the yield: 96.6%). By subjecting the dried, crude product to recrystallization from a 2:1 mixed solvent of ethanol and ethyl acetate, 59.9 g of colourless prisms (the yield: 85.0%) were obtained as the product of the process according to the present invention, the physical properties thereof being shown as follows.
(1) Melting point: 126.0 to 127.0°C (by capillary method)
(2) Elementary analytical data:

| | C(%) | H(%) |
|---|---|---|
| Found | 55.70 | 4.90 |
| Calcd. as C₁₅H₁₆O₈ | 55.56 | 4.97 |

(3) Infrared absorption spectrum (by KBr-tablet method) The spectrum is shown in Fig. 1.
(4) Nuclear magnetic resonance spectrum of ¹H:
The spectrum is shown in Fig. 2 with the following peaks:
δ (DMSO-d₆) ppm,
2.12(s) : 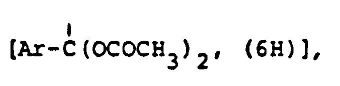 2.28(s) : [Ar-OCOCH₃, (6H)],
7.37 to 7.42(m) : [Ar-H, (3H)],
7.55(s) :

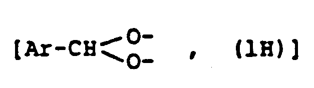

### FORMULATION EXAMPLE 1:

### Preparation of an injection

Ten parts by weight of 3,4-diacetoxybenzylidene diacetate (the present substance No. 1), 3 parts by weight of benzyl alcohol and 87 parts by weight of an aqueous physiological saline solution were mixed under heating, and the thus heated uniform mixture was sterilized to obtain an injection.

## Claims

1. A process for the preparation of 3,4- or 3,5-diacetoxybenzylidene diacetate, which process comprises reacting 3,4- or 3,5-dihydroxybenzaldehyde with (CH₃CO)₂O in the presence of concentrated sulphuric acid while stirring or shaking for a time period of from one minute to five hours.

2. A process according to claim 1, wherein the process is carried out for a time period of from two minutes to one hour.

## Patentansprüche

1. Verfahren zur Herstellung von 3,4- oder 3,5-Diacetoxybenzylidendiacetat, umfassend die Umsetzung von 3,4- oder 3,5-Dihydroxybenzaldehyd mit (CH₃CO)₂O in Gegenwart von konzentrierter Schwefelsäure unter Rühren oder Schütteln während eines Zeitraums von 1 Minute bis 5 Stunden.

2. Verfahren gemäss Anspruch 1, dadurch **gekennzeichnet,** dass das Verfahren während eines Zeitraums von 2 Minuten bis zu 1 Stunde ausgeführt wird.

## Revendications

1. Procédé pour la préparation de diacétate de 3,4- ou de 3,5-diacétoxybenzylidène, lequel procédé comprend la réaction de 3,4- ou de 3,5-diahydrorybenzaldéhyde avec du (CH₃CO)₂O en présence d'acide sulfurique concentré sous agitation ou secouement pendant une durée d'une minute à cinq heures.

2. Procédé selon la revendication 1 dans lequel le procédé est effectué pendant une durée de deux minutes à une heure.
